# EUROPEAN PATENT APPLICATION

(11) **EP 4 122 445 A1**
(43) Date of publication of application: **25.01.2023**
(21) Application number: 21306016.3
(22) Date of filing: 19.07.2021
(51) Int. Cl.: A61K 9/00, A61K 9/10, A61K 9/16, A61K 31/165, A61P 19/02, A61P 19/06

(54) **DOSAGE FORM FOR INTRA-ARTICULAR INJECTION COMPRISING COLCHICINE FOR USE IN THE TREATMENT OF CRYSTAL-AND NON-CRYSTAL ASSOCIATED ACUTE INFLAMMATORY ARTHRITIS**

(71) Applicant: PK MED, 75003 Paris (FR)
(72) Inventor: SANSON, Charles, 69007 Lyon (FR); HAYOZ, Daniel, 1752 Villars sur Glâne (FR); EA, Hang-Korng, 75018 PARIS (FR); POULIQUEN, Gauthier, 69360 TERNAY (FR)
(74) Representative: Nony

(57) **Abstract**

The present invention relates to a pharmaceutical composition under the form of a suspension comprising controlled release microparticles comprising colchicine, suitable for intra-articular injection into a joint, for use in the treatment of crystal-associated and non-crystal associated acute inflammatory arthritis, in particular a crystal-associated arthropathy attacks or flares or acute forms of tendinitis and capsulitis, wherein the microparticles have a mean particle size greater than 10 µm, wherein the time required to release 80% by weight of the colchicine in the microparticles lies between 1 day and 15 days, wherein the colchicine is present in a concentration ranging from 0.05 to 5 mg per ml of suspension and wherein the pharmaceutical composition has a volume ranging from 0.1 ml to 5 ml.

It also relates to a formulation under the form of a powder comprising controlled release microparticles comprising colchicine and a polymeric matrix, wherein the percentage weight ratio between the colchicine and the total weight of the microparticles ranges 1 to 35% by weight.

It further relates to a pharmaceutical composition under the form of a sterile and injectable suspension suitable for an intra-articular injection, obtained by mixing a formulation under the form of a powder according to the invention, with an aqueous injection vehicle.

## Description

The present invention relates to the field of the treatment of inflammatory pain in joints by colchicine. Inflammatory pain in joints aimed at in the present invention may be crystal-associated and non-crystal associated acute arthritis, in particular arthropathies attacks or flares, and acute forms of tendinitis and capsulitis and merely targets acute diseases. Colchicine is administered locally by intra articular injection *via* a controlled release dosage form.

### BACKGROUND

Inflammatory arthritis are a group of chronic auto-immune and auto-inflammatory diseases characterized by joint inflammation with symptoms such as pain, swelling, stiffness and decreased range of motion. Patients generally experience alternating acute periods with highly intense symptoms in one or several joints and periods of inactivity. Inflammatory arthritis includes several diseases and conditions such as Rheumatoid arthritis, Ankylosing spondylitis, Juvenile arthritis, Psoriatic arthritis, Lupus or Microcrystalline diseases.

In particular, the class of microcrystalline arthropathies corresponds to an inflammation of local tissue caused by the deposition of microcrystals in joints. These crystals trigger an inflammatory reaction and severe joints pain. This class of diseases includes several conditions which differ in particular in the nature of the crystals: Gout (monosodium urate - MSU), chondrocalcinosis (calcium pyrophosphate - CPP), calcifying tendinitis (calcium phosphate) and Milwaukee shoulder syndrome (calcium phosphate). In this context, the crystal-associated arthropathies are characterized by multiple, extremely painful acute attacks or flares followed by pain, disability and impaired quality of life.

Tendinitis is an inflammation of a tendon that can be secondary to several factors including traumatic, degenerative, iatrogenic i.e. drug-induced, age and inflammation, responsible of pain and disability. Tendinitis can occur at any age, but it is more common among adults who do a lot of sport or any intense cyclic tractions on the tendon. Older people are also susceptible, because the tendons tend to lose elasticity and become weaker with age. A chronic pain presentation also exists in calcifying tendinitis, as well as an acute form of calcifying tendinitis.

Capsulitis is an inflammation of a capsule generating pain and stiffness. Capsulitis frequently affects the shoulder and, in this case, may also be called frozen shoulder.

For tendinitis and capsulitis, the present invention mainly targets their acute form and for example calcifying tendinitis.

Treatment recommendations for these acute joint diseases are most often symptomatic and aim to control the pain. Generally, the first line options are mainly oral nonsteroidal anti-inflammatory drugs (NSAIDs), oral or intra-articular corticosteroids or oral colchicine (reglementary approved for gout). When it is possible and when the joints are accessible, i.e. in the case of large joints (knees, ankles, wrists, elbows and shoulders), intra-articular corticosteroid injection is considered by practitioners to avoid systemic administration and thus avoid the risk of significant side effects.

Intra-articular administration of corticosteroid, used for several decades in joint diseases in general, permits an improvement in pain and mobility, with non-lasting effects (a few weeks in the case of an immediate release form). However, the toxicological aspect of these administrations is quite problematic and also controversial. Indeed, systemic effects are mentioned for weeks following the injection including Cushing's syndrome, hyperglycemia, decreased bone density or a risk of infections. The latter being the consequence of the direct introduction of an infectious agent into the articulation or by reducing the immune response and allowing a latent tuberculosis infection to be reactivated. This type of treatment is in particular not adapted for patients at risk, i.e. for example with comorbidities, like diabetes or osteoporosis. In addition, deleterious effects are also described on local tissue as damage to articular cartilage and tendons.

Consequently, in medical practice, the frequency and dose of intra-articular corticosteroid injections are limited. For the immediate release galenic forms it is therefore recommended a maximum of three injections per year/per site and a minimum interval of three months between two injections. A first intra-articular sustained-release formulation of corticosteroid (Zilretta^{®} by Flexion Therapeutics) was approved in 2017 by the FDA for osteoarthritis, allowing continuous treatment for months. However, this product is not approved for repeated administrations.

Intra-articular administration of corticosteroids therefore does not seem to be an optimal treatment for extremely painful attacks, such as gout attack or chondrocalcinosis.

Colchicine is a drug characterized by pleiotropic effects related to its capacity to bind to tubulins and disturb microtubule polymerization, destabilizing the cytoskeleton and all the cellular processes, including cell division, migration and cell shape. Among these effects, colchicine affects the immune system and downregulates multiple inflammatory pathways, leading to a decrease in neutrophil function and migration. Colchicine is therefore used and approved in several inflammatory diseases such as Familial Mediterranean Fever, Behcet disease and Microcrystalline diseases.

Colchicine has indeed been used for a very long time for the non-specific treatment of crystal-associated arthropathies. In the past 50 years, the use of short courses of fast-acting NSAIDs and corticoids have been considered with colchicine as the oral drugs of choice for the symptomatic treatment of acute gout and pseudogout. In the article George Nuki, MB, FRCP "Colchicine: its Mechanism of Action and Efficacy in Crystal-Induced Inflammation", Current Rheumatology Reports, 2008, 10:218-227, prophylactic and therapeutic efficacy of oral administration of colchicine is reviewed. Still in said article, side effects of colchicine are extensively described, such as powerful purgative effects, bone marrow suppression, neuromyopathy and rhabdomyolysis, especially in patients with renal insufficiency as well as high mortality associated with overdosage.

Colchicine has thus an exceptionally narrow therapeutic index. Therefore, for all these reasons, the use of colchicine remains limited although its efficacy has been largely proven.

Moreover, although it is known to treat inflammation in joints, i.e. at site, by directly administering an active substance therein, it has been found that the active substance escapes relatively easily from said joint, thus reducing the therapeutic effect.

Colchicine encapsulation for sustained release has been reported in different carriers, such as in polymeric microspheres (Das, G. S. et al. "Colchicine Encapsulation within Poly(Ethylene Glycol)-Coated Poly(Lactic Acid)/Poly(ε-Caprolactone) Microspheres-Controlled Release Studies" Drug Delivery, 7: 7544, 129-138 (2000)). In this article, colchicine has been investigated in the treatment of other diseases, such as restenosis after angioplasty or vascular injury, and on this occasion, attempts of encapsulation of colchicine within poly(lactic acid)/poly(ε-Caprolactone) microspheres were performed for local delivery of colchicine. Another composition comprising biodegradable microparticles containing colchicine was further described seeking the treatment of the same pathology, i.e. restenosis, through intramural delivery, as reported in Gradus-Pizlo Irmina et al: "local delivery of biodegradable microcapsules containing colchicine or a colchicine analogue: effects on restenosis and implications of catheter-based drug delivery", Journal of The American College of Cardiology, vol.26, no. 6, pages 1549-1557. It clearly comes out that the therapeutic target is completely different from the one of the present invention. In addition, the results are not encouraging and point out toxicity risks. Indeed, signs of local toxicity were observed in the muscle layers overlying and fed by arteries infused with the microparticles containing colchicine or colchicine analogue but not control microparticles. For both disclosures, it is furthermore worth to notice that the particle size is lower than the one of the particles implemented in the present invention, as it will be apparent in the following description.

In addition, colchicine has previously been reported in US5,747,060, again for other effects than the one as directed to in the framework of the present invention, as acting on the prolongation of the duration of the anesthetic effect produced by the local administration of an anesthetic. Colchicine is thus co-administered in example 1 of said document around the sciatic nerve with bupivacaine, under an immediate release form, i.e. directly added into an aqueous solution.

WO2006/066419 discloses the same effect of colchicine with respect to the prolongation of its anesthetic effect and namely a combination of a vanilloid receptor agonist and another molecule, possibly colchicine. An example discloses a composition comprising a mixture of resiniferatoxin and colchicine injected intra-articularly for the treatment of capsulitis. Said injection is however additionally dedicated for an immediate release not seeking to control the local profile and systemic pharmacokinetics of colchicine, nor to extend its therapeutic exposure.

The use of colchicine for the treatment of crystal-associated arthropathies is well established. However, nowadays, colchicine is only commercialized under an immediate release oral dosage form, classically with a daily dosage for the treatment of the crystal-associated arthropathy around 1 mg per day. The FDA-approved dosage for the treatment of acute gout flare is 1.2 mg colchicine at the first sign of the flare followed by 0.6 mg one hour late(https://www.fda.gov/drugs/postmarket-drug-safety-information-patients-and-providers/colchicine-marketed-colcrys-information). The recommended colchicine first-line treatment for acute flare by EULAR is a loading dose of 1 mg followed 1 hour later by 0.5 mg (Richette, P. et al. 2016 updated EULAR evidence-based recommendations for the management of gout. Ann. Rheum. Dis. 76, 29-42 (2017)). However, as explained above, and illustrated herein after in more details, reaching colchicine therapeutically active dose locally is hindered by the low tolerance or high toxicity of orally administered colchicine rendering the drug prescription extremely complicated.

Colchicine was recently described in a nanoemulsion system injected into the joint of Swiss albino mice model with MSU (Monosodium Urate) crystal-induced gouty arthritis: Aboumanei et al, "intra-articular formulation of colchicine loaded nanoemulsion systems for enhanced locoregional drug delivery: In vitro characterization, 99mTc coupling and in vivo biodistribution studies", DOI: 10.1080/03639045.2021.1934865. The biodistribution pattern of ^{99m}Tc-ColNE-5 as well as ^{99m}Tc-Col solution (^{99m}TcColS) was studied. However, said disclosure is limited to an observation of a radiolabeled colchicine which is thus a different molecule from colchicine itself and within a period not exceeding 24 hours. It is well known that labelling with radionuclide like ^{99m}Tc can have a strong impact on the pattern of biodistribution of the labelled entity due to modification of its charge, lipophilicity and stability (Decristoforo et al. "The influence of chelator on the pharmacokinetics of 99mTc-labelled peptides." Q. J. NUCL. MED. 46 3 (2002): 195-205). Moreover, said article is silent on the technical problem of narrow therapeutic index as explained above and very specific to the colchicine, i.e. the maximal systemic concentration to be avoided for toxicity reasons. Said article is thus also silent on microparticulate systems with higher sizes and targeted dosages able to reach effective local concentration for combatting the acute forms of the considered pathologies, with no toxicity at a systemic level. In other words, no colchicine controlled release profile suitable for human healing is revealed in said article, and even more particularly for the treatment of a crystal-associated or non-crystal associated acute inflammatory arthritis, in particular effective in treating non-crystal associated acute arthritis, such as Rheumatoid arthritis, Ankylosing spondylitis, Juvenile arthritis, Psoriatic arthritis, Lupus or in treating crystal-associated arthropathies attacks or flares, such as Gout, Chondrocalcinosis, calcifying tendinitis and Milwaukee shoulder syndrome.

IV injection of colchicine is considered effective in the treatment of acute gout attack with a dose not exceeding 2-3 mg per single injection (Nuki, G. Colchicine: Its mechanism of action and efficacy in crystal-induced inflammation. *Curr. Rheumatol. Rep.* **10**, 218-227 (2008)). However even if GI side effects can be avoided using IV administration, severe toxicity was associated with inappropriate dosing (Wallace SL, S. J. Review: systemic toxicity associated with the intravenous administration of colchicine-guidelines for use. J Rheumatol. 15, 495-9 (1988), Bonnel, R. A., Villalba, M. L., Karwoski, C. B. & Beitz, J. Deaths associated with inappropriate intravenous colchicine administration. J. Emerg. Med. 22, 385-387 (2002)). This led authorities to eliminate the use of IV colchicine for acute gout treatment (Zhang, W. et al. EULAR evidence-based recommendations for gout. Part II: Management. Report of a task force of the EULAR Standing Committee for International Clinical Studies Including Therapeutics (ESCISIT). Ann. Rheum. Dis. 65, 1312-1324 (2006)). Attempts of i.v. injection of colchicine have furthermore largely discouraged the man skilled in the art to envision injection of colchicine, as reported in E.Niel et al, "Colchicine today", Joint Bone Spine 73 (2006) 672-678.

Therefore, it remains a need to develop novel dosage forms, route of administrations and targeted dosage for colchicine, both for obviating the side-effects, in particular observed during systemic administration, and improving the efficacy by optimizing the available dose of colchicine, in particular for extending its local duration of action at the painful site, in particular by increasing the local concentration, in particular for the treatment of acute crisis of crystal-associated inflammatory arthritis and non-crystal associated inflammatory arthritis.

The invention has for purpose to meet the needs, thereby both obtaining increased advantage from its action against the inflammation and restricting the level of any side effects.

One of the advantages of the present invention is to provide a new mean to achieve a particular colchicine release profile which respects an adequate ratio between the local concentration of colchicine and the systemic concentration thereof, suitable for offering the best therapeutic action locally and at the same time avoiding potential systemic toxicity.

Due to the reduced side-effects obtained by the administration of the pharmaceutical composition according to the present invention, it is furthermore particularly suitable for people suffering from chronic comorbidities (Hypertension, Chronic Kidney diseases, Diabete, Heart diseases, Immunosuppression...) fairly prevalent conditions in microcrystalline joint diseases (50% of Gout patients having at least 3 comorbidities). Indeed, the management of microcrystalline joint diseases patients having multiple comorbidities, remains a challenge for medical practitioners due to frequent and major contraindications to either Colchicine, NSAIDS or Corticosteroids. In comparison to the existing oral colchicine treatment, the present invention, in addition to avoid gastrointestinal side effects, prevents major toxicity risks for patients suffering from renal or hepatic impairments or for co-medicated patients with CYP3A4/PGP drug inhibitors, which are fairly prevalent medications.

### SUMMARY OF THE INVENTION

According to a first aspect, the invention relates to a pharmaceutical composition under the form of a suspension comprising controlled release microparticles comprising colchicine, suitable for intra-articular injection into a joint, for use in the treatment of a crystal and non-crystal-associated acute inflammatory arthritis or acute forms of tendinitis and capsulitis, wherein the microparticles have a mean particle size greater than 10 µm, wherein the time required to release 80% by weight of the colchicine in the microparticles lies between 1 day and 15 days, wherein the colchicine is present in a concentration ranging from 0.05 to 5 mg per ml of suspension and wherein the pharmaceutical composition has a volume ranging from 0.1 ml to 5 ml.

According to a second main embodiment, the invention relates to a formulation under the form of a powder comprising controlled release microparticles comprising colchicine and a polymeric matrix, wherein the microparticles have a mean particle size equal or greater than 10 µm, and wherein the polymeric matrix comprises at least a poly(lactic-co-glycolic acid) copolymer, at least a poly(caprolactone) or at least a mixture of poly(lactic-co-glycolic acid) copolymer and poly(caprolactone), wherein the percentage weight ratio between the colchicine and total weight of the microparticles ranges from 1 to 35% by weight, in particular from 2 to 30% by weight, more particularly between 5 and 25% by weight, and even more particularly between 10 and 20% by weight.

According to a third main embodiment, the invention relates to a pharmaceutical composition under the form of a sterile and injectable suspension suitable for an intra-articular injection, obtained by mixing a formulation under the form of a powder as described above, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

The pharmaceutical composition suitable for an intra-articular injection as described herein after is effective in treating crystal-associated and non-crystal associated acute inflammatory arthritis, in particular effective in treating non-crystal associated acute arthritis, such as Rheumatoid arthritis, Ankylosing spondylitis, Juvenile arthritis, Psoriatic arthritis, Lupus or in treating crystal-associated arthropathies attacks or flares, such as gout, chondrocalcinosis, calcifying tendinitis and Milwaukee shoulder syndrome with minimal long-term side effects.

Said pharmaceutical composition suitable for an intra-articular injection is suitable for local administration by injection into a site at or near the site of pain.

### DETAILED DESCRIPTION OF THE INVENTION

As apparent from the BACKGROUND paragraph above, the prescription of colchicine is nowadays delicate as only oral compositions are available and high risk of toxicity have been observed, such as gastrointestinal symptoms and neuromuscular involvement. Risks linked to interaction with other active agents have further been reported. And again, toxicity constraints frankly have discouraged the local injection for decades.

Indeed, it has been established in the literature that effective local dose of 0.015 mg/kg to 0.030 mg/kg colchicine is recommended, that at 0.1 mg/kg the toxicity limit is reached, and that at 0.8 mg/kg lethal dose is reached (E.Niel et al, "Colchicine today", Joint Bone Spine 73 (2006) 672-678). It is thus concluded that the narrow therapeutic margin for colchicine is a source of concern for prescribing physicians.

The inventors have found that compared to the treatment of oral colchicine or intra-articular corticosteroids in joint pain diseases, the intra-articular injection of a pharmaceutical composition under the form of a suspension for a controlled release of colchicine has at least equivalent and even higher efficiency, when the time required to release 80% by weight of the colchicine in the microparticles lies between 1 day and 15 days and when the colchicine is present in a concentration ranging from 0.05 to 5 mg per ml of suspension and wherein the pharmaceutical composition has a volume ranging from 0.1 ml to 5 ml.

The diseases for which the use of said pharmaceutical composition is dedicated is very intense. Thus, having treatments perfectly adapted for achieving the most appropriate dosage is crucial.

The use or method of treatment according to the present invention also has the advantage of reducing the significant toxicity risks, linked to drug interaction with colchicine when administered *via* a systemic route, and in particular in the elderly, or patients with kidney or liver failure. In comparison to intra-articular corticosteroids, this treatment also has the advantage to avoid systemic effects such as Cushing's syndrome, hyperglycemia, hypertension, decreased bone density or even the risk of infections. In addition, it decreases the risk of deleterious effects on local tissue as damage to articular cartilage and tendons.

### Definitions

As used herein, the term "patient" refers to either an animal, such as a valuable animal for breeding, company or preservation purposes, or preferably a human or a human child, which is afflicted with, or has the potential to be afflicted with one or more diseases and conditions described herein.

In particular, as used in the present application, the term "patient" refers to a mammal, including a non-human mammal such as a rodent, cat, dog, or primate, or a human; preferably said subject is a human and also extends to birds.

The identification of those patients who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term "treating" or "treatment", as used herein, means reversing, alleviating, inhibiting the progress of non-crystal associated acute inflammatory arthritis, such as Rheumatoid arthritis, Ankylosing spondylitis, Juvenile arthritis, Psoriatic arthritis, Lupus or in treating crystal-associated acute inflammatory arthritis, in particular crystal-associated arthropathies attacks or flares, such as gout, chondrocalcinosis, calcifying tendinitis and Milwaukee shoulder syndrome, and acute forms of tendinitis and capsulitis.

As used herein, an "effective amount" refers to an amount of a compound of the present invention which is effective in reducing, eliminating, treating or controlling the symptoms of the herein-described diseases and conditions, *i.e.* crystal-associated and non-crystal associated acute inflammatory arthritis, in particular non-crystal associated acute arthritis, such as Rheumatoid arthritis, Ankylosing spondylitis, Juvenile arthritis, Psoriatic arthritis, Lupus or in treating and crystal-associated arthropathies attacks or flares, such as gout, chondrocalcinosis, calcifying tendinitis and Milwaukee shoulder syndrome, and acute forms of tendinitis and capsulitis. The term "controlling" is intended to refer to all processes wherein there may be a slowing, interrupting, arresting, or stopping of the progression of the diseases and conditions described herein, but does not necessarily indicate a total elimination of all diseases and condition symptoms.

The term "treatment-effective amount" refers to a concentration of compound that is effective in treating crystal-associated and non-crystal associated acute inflammatory arthritis, in particular non-crystal associated acute arthritis, such as Rheumatoid arthritis, Ankylosing spondylitis, Juvenile arthritis, Psoriatic arthritis, Lupus or in treating crystal-associated arthropathies attacks or flares, *e.g.* leads to a reduction in inflammatory pain, following examination when administered after initiation of the inflammatory pain has occurred.

As used herein, the term "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

The term "pharmaceutically acceptable carrier, adjuvant, or vehicle" may refer to any pharmaceutically acceptable excipient, such as a non-toxic carrier, adjuvant, or vehicle that does not destroy the pharmacological activity of the compound with which it is formulated.

Within the framework of the present invention, the term "painful site" or "site of the pain" means the joint area causing pain where the product can be injected. It can be the knee joint, the hip joint, metacarpophalangeal joints, the shoulder joint, the wrist joint, the elbow joint, the metatarsophalangeal joint, the ankle joint, the vertebral joint and the midfoot joints.

The terms "controlled release composition" or "controlled release microparticle" mean the composition or the microparticle allow a release into the body of a specified amount over a specified period of time of an active ingredient, namely a specific pharmacokinetic profile. The term "controlled release" encompasses all the type of releases that are modified in comparison to an immediate release. In other words, the term "controlled release" is equivalent to "modified release" and encloses extended releases as defined herein after as well as delayed release and pulse releases.

The terms "extended release", "prolonged release" and "sustained release" are considered equivalent in the framework of the present invention. This type of release means that the release is prolonged over time in comparison to an immediate release, i.e. it means that the active ingredient is released slowly over time, allowing a less frequent intake of the drug for the patient. In other words, the active ingredient is progressively released over a specific period of time, generally aiming at less side effects as well by decreasing the maximal concentration.

The term "drug loading content" means the mass ratio of the drug in the microparticles to the mass of microparticles.

The term "mean particle size" or D50 means the particle diameter in microns that splits the particle volume distribution with half above and half below this diameter.

A controlled or modified release may of course result from the combination of an immediate release and a controlled release.

### COLCHICINE

Colchicine is an alkaloid extracted from the corm of the meadow saffron or autumn crocus (*Colchicum autumnale*)*.* Its IUPAC name is N-[(7S)-1,2,3,10-tetramethoxy-9-oxo-6,7-dihydro-5H-benzo[a]heptalen-7-yl]acetamide.(CAS 64-86-8)

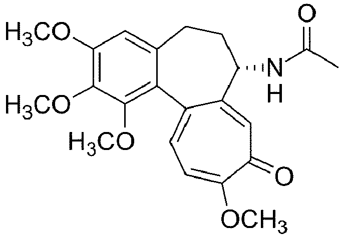

Colchicine may exist in racemic of enantiomeric form. All said forms are encompassed within the scope of the invention.

Moreover, it may exist in various crystalline forms depending on the solvent used for obtaining them. Chloroform, dichloromethane, ethyl acetate, benzene or any other suitable solvent may be cited among possible solvents. All such crystalline forms also form part of the present invention.

It is mainly known as gout suppressant and as an anti-inflammatory agent *via* its tubulin interaction activity. It is administered by the oral route although intravenous administration was also attempted and then abandoned for excessive toxicity reasons.

### THERAPEUTIC USE AND METHODS

In one embodiment, the pharmaceutical composition suitable for an intra-articular injection as described herein after delivers colchicine in a dose and in a controlled release manner such that the concentration level of colchicine at the treated articular site is above the systemic dosage classically obtained after an oral administration, i.e. above 5 nM (2 ng/mL), and the systemic concentration level stays below the value of 15 nM (6 ng/mL). (Terkeltaub, R. A. et al. High versus low dosing of oral colchicine for early acute gout flare: Twenty-four-hour outcome of the first multicenter, randomized, double-blind, placebo-controlled, parallel-group, dose-comparison colchicine study. Arthritis Rheum. 62, 1060-1068 (2010)).

As above mentioned, the controlled release form is administered locally to treat pain and inflammation in the joints. Local administration of the formulation may occur by injection into the intra-articular space or peri-articular space at or near the site of a patient's pain, including the metatarsophalangeal joints, the metacarpophalangeal joints, the knee joint, the shoulder joint, the wrist joint, the elbow joint, the ankle joint, the hip joint, a vertebral joint and the midfoot joints. The midfoot is the anterior part of the foot that sits between the hindfoot and forefoot. It is composed of the cuboid, navicular and three cuneiform bones. The mid-tarsal and the tarsometatarsal joints join the midfoot to the hindfoot and the forefoot respectively. The midfoot is a complex and composite area where observed oedema inflammation is generally diffuse.

In one embodiment, the present invention is dedicated to the treatment of pain in joints, in particular crystal-associated and non-crystal associated acute inflammatory arthritis, more particularly selected from non-crystal associated acute arthritis, such as Rheumatoid arthritis, Ankylosing spondylitis, Juvenile arthritis, Psoriatic arthritis, Lupus or in treating and crystal-associated arthropathies attacks or flares, such as gout, chondrocalcinosis, calcifying tendinitis and Milwaukee shoulder syndrome, and acute forms of tendinitis and capsulitis.

In one embodiment, the composition suitable for an intra-articular injection, is dedicated to the treatment of gout attack.

In one embodiment, the composition suitable for an intra-articular injection is dedicated to the treatment of chondrocalcinosis.

In one embodiment, the composition suitable for an intra-articular injection is dedicated to the treatment of acute and chronic calcifying tendinitis.

In one embodiment, the composition suitable for an intra-articular injection is dedicated to the treatment of Milwaukee shoulder syndrome.

In one embodiment, composition suitable for an intra-articular injection is dedicated to the treatment of tendinitis.

In one embodiment, composition suitable for an intra-articular injection is dedicated to the treatment of capsulitis.

The administration may be performed by a single injection or as sequential injections provided that the time required to release 80% by weight of the colchicine in the microparticles lies between 1 day and 15 days, the colchicine being present in a concentration ranging from 0.05 to 5 mg per ml of suspension and the pharmaceutical composition having a volume ranging from 0.1 ml to 5 ml. In other words, the single or sequential injections, preferably the single injection, provides locally a colchicine weight ranging between 0.05 and 7.5 mg of colchicine.

In one embodiment, the injection is performed a plurality of times (two times, three times, four times or more). In this embodiment, each subsequent time the injection is performed, it is performed after a suitable period has lapsed since the preceding time the injection was performed. This suitable time can be, for example, two weeks, one month, two months, three months, four months, five months, six months, one year, or longer.

According to a particular embodiment, the colchicine is present in a concentration ranging from 0.1 to 4 mg per ml of suspension, in particular from 0.25 to 2.5 mg per ml of suspension, and more particularly from 0.5 to 1.5 mg per ml of suspension.

In a particular embodiment, the same pharmaceutical composition under the form of a suspension may be used for intra-articular injection to any painful joint. The amount of pharmaceutical composition to be injected may however be adapted to the targeted joint. This is one of the advantages of the invention, which may with a single concentration offer the adapted amount of colchicine for each site of application by adapting the corresponding needed volume.

In one embodiment, injections may be performed simultaneously in two or more different painful joints.

For example, the volume of pharmaceutical composition under the form of a suspension according to the present invention that may be injected to the shoulder may vary between 2 and 5 ml.

The volume of pharmaceutical composition under the form of a suspension according to the present invention that may be injected to the metatarsophalangeal joints, for example the toes, may vary between 0.1 and 0.5 ml, typically is 0.25 ml.

The volume of pharmaceutical composition under the form of a suspension according to the present invention that may be injected to the metacarpophalangeal joints, for example the fingers, may vary between 0.1 and 0.5 ml, typically is 0.25 ml.

The volume of pharmaceutical composition under the form of a suspension according to the present invention that may be injected to a vertebral joint may vary between 1 and 2 ml.

The volume of pharmaceutical composition under the form of a suspension according to the present invention that may be injected to the midfoot joints may vary between 0.1 and 1 ml.

The volume of pharmaceutical composition under the form of a suspension according to the present invention that may be injected to the knee, may vary between 2 and 5 ml.

The volume of pharmaceutical composition under the form of a suspension according to the present invention that may be injected to the wrist may vary between 0.5 and 1 ml.

The volume of pharmaceutical composition under the form of a suspension according to the present invention that may be injected to the elbow may vary between 2 and 5 ml.

The volume of pharmaceutical composition under the form of a suspension according to the present invention that may be injected to the ankle may vary between 1 and 3 ml.

The volume of pharmaceutical composition under the form of a suspension according to the present invention that may be injected to the hip may vary between 2 and 5 ml.

In one embodiment, the pharmaceutical composition for use according to the present invention may be characterized by its volume, which may be adapted to the joint, and namely may range from 2 to 5 ml for the shoulder, from 1 to 2 ml for a vertebral joint, from 0.1 to 1 ml for the midfoot joints, from 0.1 to 0.5 ml for the metatarsophalangeal joints, from 0.1 to 0.5 ml for the metacarpophalangeal joints, from 2 to 5 ml for the knee joint, from 0.5 to 1 ml for the wrist joint, from 2 to 5 ml for the elbow joint, from 1 to 3 ml for the ankle joint and from 2 to 5 ml for the hip.

It should also be understood that a specific dosage and treatment regimen for any particular patient will depend upon a variety of factors, including the age, body weight, general health, sex, diet, time of administration, drug combination, and the judgment of the treating physician and the severity of the particular disease being treated.

In one embodiment, the controlled release is suitable for reaching an articular colchicine maximum concentration (Cmax) ranging from 5 nM (2ng/mL) to 5 µM (2 µg/mL).

According to a preferred embodiment, the injection of the pharmaceutical composition of the present invention allows reaching a systemic concentration of colchicine which does not exceed 15 nM (6 ng/mL) during the whole duration of the treatment.

Thus, injection of the controlled release pharmaceutical composition according to the invention can be able to achieve a maximum concentration (Cmax) of colchicine locally in the ranges as given above, at a rate that allows avoiding or at least decreasing the here above mentioned side effects, in particular reaching a maximum systemic concentration of colchicine of 15 nM (6 ng/mL) during the whole duration of the treatment, at least after the optional burst release as described herein after.

The requested concentration to be injected into the painful joint can depend on the type of painful joint, in particular depending on its size. Requested concentration of colchicine may indeed be higher for small joints than for medium-large joints. Table 1 as follows gathers typical dosage that may be implemented in the framework of the present invention.

**Table 1:**

| | **Joint** | **Volume (ml)** | **Drug concentration (mg/ml)** | **Dose range (mg)** |
|---|---|---|---|---|
| **Small joints** | | | | |
| | metatarsophalangeal | 0.1-0.5 | 0.5-5 | 0.05-2.5 |
| | metacarpophalangeal | 0.1-0.5 | 0.5-5 | 0.05-2.5 |
| | wrist | 0.5-1 | 0.5-5 | 0.25-5 |
| | midfoot | 0.1-1 | 0.5-5 | 0.05-5 |

| **Medium/large joints** | | | | |
|---|---|---|---|---|
| | vertebral | 1-2 | 0,05-1,5 | 0,05-3 |
| | ankle | 1-3 | 0.05-1.5 | 0.05-4.5 |
| | knee | 2-5 | 0.05-1.5 | 0.1-7.5 |
| | hip | 2-5 | 0.05-1.5 | 0.1-7.5 |
| | shoulder | 2-5 | 0.05-1.5 | 0.1-7.5 |
| | elbow | 2-5 | 0.05-1.5 | 0.1-7.5 |

In one embodiment, the pharmaceutical composition as used in the present invention, which is in particular administered in one intra-articular injection, and which is dedicated to the treatment of the acute phase of crystal-associated inflammatory arthritis or non-crystal associated inflammatory arthritis or acute forms of tendinitis and capsulitis, comprises between 0.05 to 2.5 mg of colchicine per injection for a small joint selected from metatarsophalangeal joint or metacarpophalangeal joint.

In another embodiment, the pharmaceutical composition as used in the present invention, which is in particular administered in one intra-articular injection, and which is dedicated to the treatment of the acute phase of crystal-associated inflammatory arthritis or non-crystal associated inflammatory arthritis or acute forms of tendinitis and capsulitis, comprises between 0.25 and 5 mg of colchicine per injection for a wrist joint.

In another embodiment, the pharmaceutical composition as used in the present invention, which is in particular administered in one intra-articular injection, and which is dedicated to the treatment of the acute phase of the crystal-associated inflammatory arthritis or non-crystal associated inflammatory arthritis or acute forms of tendinitis and capsulitis, comprises between 0.05 and 5 mg per injection for midfoot joints.

In another embodiment, the pharmaceutical composition as used in the present invention, which is in particular administered in one intra-articular injection, and which is dedicated to the treatment of the acute phase of the crystal-associated inflammatory arthritis or non-crystal associated inflammatory arthritis or acute forms of tendinitis and capsulitis, comprises between 0.05 and 4.5 mg per injection for an ankle joint.

In another embodiment, the pharmaceutical composition as used in the present invention, which is in particular administered in one intra-articular injection, and which is dedicated to the treatment of the acute phase of the crystal-associated inflammatory arthritis or non-crystal associated inflammatory arthritis or acute forms of tendinitis and capsulitis, comprises between 0.1 and 7.5 mg per injection for a joint selected from a knee joint, a hip joint, a shoulder joint and an elbow joint.

According to one embodiment, the pharmaceutical composition as used in the present invention, which is in particular administered in one intra-articular injection, and which is dedicated to the treatment of the acute phase of the crystal-associated inflammatory arthritis or non-crystal associated inflammatory arthritis or acute forms of tendinitis and capsulitis, comprises between 0.05 to 7.5 mg, in particular 0.05 and 6 mg of colchicine, more particularly between 0.05 and 4 mg, for example between 0.05 and 3 mg. In one embodiment, the pharmaceutical composition as used in the present invention may for example comprise between 0.2 and 5 mg of colchicine, in particular between 0.2 and 4 mg of colchicine, more particularly between 0.2 and 3 mg of colchicine. In one embodiment, the pharmaceutical composition as used in the present invention may for example comprise between 0.5 and 5 mg of colchicine, in particular between 0.5 and 4 mg of colchicine, more particularly between 0.5 and 3 mg of colchicine. In one embodiment, the pharmaceutical composition as used in the present invention may for example comprise between 1 and 5 mg of colchicine, in particular between 1 and 4 mg of colchicine.

In one embodiment, the pharmaceutical composition may be suited for maintaining a therapeutically acceptable concentration of colchicine in the painful joint during 1 to 9 days, 1 to 8 days, 1 to 7 days, 1 to 6 days, and for example during 5 days, 6 days, 7 days, 8 days, 9 days or 10 days, depending on the nature of the pathology to be treated.

In one embodiment, the pharmaceutical composition for use according to the invention, is characterized by the fact that the time required to release 80% by weight of the colchicine in the microparticles lies between 1 day and 10 days, after 1 day to 7 days, after 1 day to 5 days, and for example after 1 day, 2 days, 3 days, 4 days, 5 days, 6 days, 7 days, 8 days, 9 days or 10 days.

In one embodiment, the pharmaceutical composition as used in the present invention may be administered during 5 to 10 days and it comprises between 1 to 7.5 mg of colchicine, between 2 to 6 mg of colchicine or between 3 to 5 mg of colchicine.

In one embodiment, the pharmaceutical composition as used in the present invention may be administered during 6 to 9 days and it comprises between 2 to 7.5 mg or colchicine, in particular between 3 to 5 mg of colchicine.

It is apparent that the dosage of colchicine may be reduced in some embodiments by 10%, 20%, 30%, 40% and even more for the treatment of one acute phase of crystal-associated inflammatory arthritis or non-crystal associated inflammatory arthritis or acute forms of tendinitis and capsulitis.

According to one particular embodiment, the treatment may be renewed when another acute crises arises under the same conditions and posology.

According to one embodiment, and more particularly for the treatment of acute arthritis associated with the presence of microcrystals or non-crystal associated inflammatory arthritis, and more particularly gout attacks, chondrocalcinoses and acute calcifying tendinitis, the pharmaceutical composition may be suited for providing an initial articular colchicine concentration.

The pharmaceutical composition may or may not exhibit a burst release. In the framework of the present invention a "burst release" means that an initial bolus of drug is released before the release rate reaches a stable profile, immediately upon placement in the release medium, i.e. in the present invention immediately after the injection into the articulation.

In any case, the length of burst release, may be between 0 and 1 day, for example between the beginning of day 1 through the end of day 1, and in particular is between 0 and 6 hours, between 0 and 3 hours, and even more particularly is between 0 and 2 hours.

Thus, when a burst release occurs, upon administration, the pharmaceutical composition according to the present invention may provide an initial release of colchicine at the site of administration, for example, in the intraarticular space and/or peri-articular space. Once the initial release of colchicine has subsided, the controlled release of the colchicine microparticle formulation continues to provide therapeutic (e.g., intra-articular and/or peri-articular) concentrations of colchicine to combat inflammation and/or pain for an additional period of therapy following administration.

According to another aspect, the invention relates to a method for the treatment of crystal-associated and non-crystal associated acute inflammatory arthritis, in particular a non-crystal associated acute arthritis, such as Rheumatoid arthritis, Ankylosing spondylitis, Juvenile arthritis, Psoriatic arthritis, Lupus or a crystal-associated arthropathy attacks or flares or acute forms of tendinitis and capsulitis comprising at least the administering by injection to a painful joint to a patient in need thereof of a pharmaceutical composition comprising controlled release microparticles comprising an effective amount of colchicine, suitable for intra-articular injection into a joint, wherein the microparticles have a mean particle size greater than 10 µm, wherein the time required to release 80% by weight of the colchicine in the microparticles lies between 1 day and 15 days, wherein the colchicine is present in a concentration ranging from 0.05 to 5 mg per ml of suspension and wherein the pharmaceutical composition has a volume ranging from 0.1 ml to 5 ml.

Still according to another aspect, the invention relates to a method for the treatment of crystal-associated and non-crystal associated acute inflammatory arthritis, in particular a non-crystal associated acute arthritis, such as Rheumatoid arthritis, Ankylosing spondylitis, Juvenile arthritis, Psoriatic arthritis, Lupus or a crystal-associated arthropathy attacks or flares or acute forms of tendinitis and capsulitis comprising at least the administration by injection into the joints of a patient in need thereof of at least an efficient amount of colchicine for a controlled release.

Herein is further provided a method for the treatment of crystal-associated and non-crystal associated acute inflammatory arthritis, in particular of crystal-associated arthropathy attacks or flares or acute forms of tendinitis and capsulitis comprising at least the steps of:
- preparing and/or providing a pharmaceutical composition under the form of a sterile and injectable suspension by mixing a formulation under the form of a powder as described herein after, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder, and
- injecting said pharmaceutical composition into a painful joint of a patient in need thereof, wherein the volume to be injected is adapted to the injection site.

Herein is further provided a kit or article of manufacture comprising, in separate compartments (i) an aqueous injection vehicle and (ii) a powder as described herein after, wherein excipient selected from a group consisting of a tonicity-enhancing agent, a wetting-agent, a viscosity-enhancing agent, a density-enhancing agent or a mixture thereof may be present in the aqueous injection vehicle or in the powder, for preparing a pharmaceutical composition suitable for an intra-articular injection.

In said embodiment, the kit or article of manufacture may be under the form of a prefilled syringe or medical device, for example comprising a dual chamber. In said embodiment, the two compartments or dual chamber may allow the mixture of the aqueous injection vehicle and the powder as described herein after by all means known to the man skilled in the art. Still in said embodiment, said means may take the form of a pierceable membrane or destructible diaphragm, for example through a pressure the user may exert.

In said embodiment, the kit or article of manufacture may additionally contain a label instructing the user to introduce the obtained pharmaceutical composition into a subject's joint.

### CONTROLLED RELEASE MICROPARTICLES COMPRISING COLCHICINE

The controlled release microparticles comprising colchicine may take the form of various microparticles, such as (i) microparticles comprising a polymeric matrix or (ii) multivesicular liposomes.

According to one embodiment, the microparticles have a mean particle size lower than 100 µm, in particular lower than 80 µm, and more particularly lower than 50 µm, for example between 10 and 50 µm or between 15 and 40 µm.

It is understood that these ranges refer to the mean size of all microparticles in a given population. The size of any given individual microparticle could be within a standard deviation above or below the mean size.

Within the context of the present invention, a "microparticle" means a particle of any shape and made of any material, in particular suitable for the injection into the human body within a pharmaceutical composition, and in particular into the articulations or joints, having a mean particle size greater than 10 µm and less than 100 µm.

The microparticles suitable for the pharmaceutical composition used according to the present invention may be selected form various types of microparticles such as microspheres, microparticle matrices, microsphere matrices, microcapsules, rods, wafers, pills fibers and pellets.

### (i) MICROPARTICLES COMPRISING A POLYMERIC MATRIX

The polymeric matrix may be chosen from various polymers suitable for obtaining the controlled release microparticles. Such polymeric matrix is nontoxic to the human body.

According to one embodiment, the polymer forming the polymeric matrix is biodegradable.

Within the context of the present invention, a "biodegradable" material, refers to a material which degrades enzymatically or hydrolytically and for which there is proof that the degradation products are integrated into biomass and/or eliminated from the organism by metabolism or renal filtration.

The nontoxic and biodegradable polymer may be natural or synthetic.

According to one embodiment, the polymeric matrix of the microparticles used in the pharmaceutical composition used according to the present invention comprises at least a poly(lactic-co-glycolic acid) copolymer or PLGA.

According to said embodiment, the polymeric matrix may comprise PLGA in an amount of greater than 70% by weight, with respect to the total weight of the polymeric matrix, in particular greater than 80% by weight, and even more particularly greater than 90% by weight.

According to another embodiment, the polymeric matrix of the microparticles used in the pharmaceutical composition used according to the present invention comprises at least a poly(caprolactone) (PCL).

Suitable polymers are not limited to those commercially available and known as RESOMER (Evonik Industries AG, Germany), LACTEL (Durect, USA), PURASORB (Corbion NV, The Netherlands), Viatel (Ashland, USA).

Examples of suitable polymers are listed in Table 2.

**Table 2:**

| **Examples of suitable polymers** | | | |
|---|---|---|---|
| **Product name** | **Polymer** | **End group** | **Producer** |
| Resomer RG 502H | Poly(D,L-lactide-co-glycolide) 50:50 | acid | Evonik |
| Resomer RG 502 | Poly(D,L-lactide-co-glycolide) 50:50 | ester | Evonik |
| Resomer RG 503H | Poly(D,L-lactide-co-glycolide) 50:50 | acid | Evonik |
| Resomer RG 503 | Poly(D,L-lactide-co-glycolide) 50:50 | ester | Evonik |
| Lactel B6013-1 | Poly(D,L-lactide-co-glycolide) 50:50 | acid | Durect |
| Lactel B6017-1 | Polv(D,L-lactide-co-glvcolide) 50:50 | ester | Durect |
| Lactel B6010-1 | Poly(D,L-lactide-co-glycolide) 50:50 | ester | Durect |
| Purasorb PDLG 5002A | Polv(D,L-lactide-co-glvcolide) 50:50 | acid | Corbion |
| Purasorb PDLG 5002 | Poly(D,L-lactide-co-glycolide) 50:50 | ester | Corbion |
| Purasorb PDLG 5004A | Polv(D,L-lactide-co-glvcolide) 50:50 | acid | Corbion |
| Purasorb PDLG 5004 | Poly(D,L-lactide-co-glycolide) 50:50 | ester | Corbion |
| Viatel | Poly(D,L-lactide-co-glycolide) 50:50 | acid/ester | Ashland |

According to said embodiment, the polymeric matrix may comprise PCL in an amount of greater than 70% by weight, with respect to the total weight of the polymeric matrix, in particular greater than 80% by weight, and even more particularly greater than 90% by weight.

According to another embodiment, the polymeric matrix of the microparticles used in the pharmaceutical composition used according to the present invention comprises a mixture of at least a PCL and at least a PLGA.

In a particular embodiment, the pharmaceutical composition for use according to the present invention, is characterized by the microparticles, which are microparticles comprising colchicine and a polymeric matrix, wherein the polymeric matrix comprises at least a poly(lactic-co-glycolic acid) copolymer, at least poly(caprolactone) or at least a mixture of poly(lactic-co-glycolic acid) copolymer and poly(caprolactone).

In one embodiment, the polymeric matrix comprises poly(lactic-co-glycolic acid), copolymer poly(caprolactone) or a mixture of poly(lactic-co-glycolic acid) copolymer and poly(caprolactone) in an amount of greater than 70% by weight, with respect to the total weight of the polymeric matrix, in particular greater than 80% by weight, and even more particularly greater than 90% by weight.

According to said embodiment, the polymeric matrix may comprise a mixture of PCL and PLGA in an amount of greater than 70% by weight, with respect to the total weight of the polymeric matrix, in particular greater than 80% by weight, and even more particularly greater than 90% by weight.

The polymeric matrix may comprise one or more additional polymer(s), copolymer(s) or mixture thereof. Said additional polymer(s), copolymer(s) or mixture thereof may be present in the polymer matrix in an amount ranging from 0 to 30% by weight, in particular from 0 to 20% by weight, and more particularly from 0 to 10% by weight, with respect to the total weight of the polymeric matrix.

Non-limiting examples of suitable additional polymers or copolymers include poly(lactide) or PLA, poly(glycolide) or PGA, poly(lactide-co-caprolactone), poly(ethylene glycol), poly(ethylene oxide) or PEO, PLGA-b-PEO-b-PLGA, PLGA-b-PEO, polyhydroxyalkanoates, poly(hydroxybutyrate), poly(trimethylene carbonate), poly(dioxanone), poly(valerolactone), poly(alpha- hydroxy acids), poly(lactones), poly(amino acids), polyanhydrides, poly(orthoester), poly(acetals), polyurethanes, polythioesters, polyphosphoesters, poly(ester-co-amide), poly(vinyl alcohol) or PVA, PVA-g-PLGA, poly (ether ester) multiblock copolymers, polyvinyl pyrrolidone, poly(methacrylate), PEO-PPO-PEO (pluronics), gelatin, heparin, chondroitin sulfate; polysaccharides such as alginates, starch, chitosan and dextrans and any combinations thereof.

According to one embodiment, the drug loading content or percentage weight ratio between the colchicine and total weight of the microparticles ranges from 1% to 35%, in particular from 2 to 30%, even more particularly from 5 to 25%, for example from 10 to 20%.

In one embodiment, the controlled release microparticles according to the present invention are PLGA microspheres. In other words, in said embodiment, the polymeric matrix does not comprise any additional polymer or copolymer.

When PLGA copolymers are implemented as a polymer matrix, they can have a wide range of molecular weights and monomer ratios of lactic to glycolic acid, in particular of 75:25 to 50:50, and even more particularly of 50:50. Any suitable method known in the art of making the polymer can be used and the molecular weights may typically range from 10 to 150 kDa, in particular from 10 to 80 kDa, and more particularly from 10 to 50 kDa.

According to a further particular embodiment, the polymer forming the polymeric matrix comprises PLGA, for example in an amount of 100% by weight, with respect to the total weight of the polymeric matrix, and the PLGA is defined by:
- A molecular weight between 10 and 80 kDa, and
- A lactide:glycolide molar ratio of 75:25 to 50:50.

According to a more particular embodiment, the polymer forming the polymeric matrix comprises PLGA, for example in an amount of 100% by weight, with respect to the total weight of the polymeric matrix, and the PLGA is defined by:
- A molecular weight between 10 and 50 kDa, and
- A lactide:glycolide molar ratio of 50:50.

According to one embodiment, PLGA is either carboxylic or ester endcapped, and is in particular carboxylic endcapped.

According to another embodiment, microparticle matrix can comprise, and even may consist in, a blend of two PLGA copolymers, in particular one of low molecular weight and one of high molecular weight.

The microparticle may further comprise pharmaceutically acceptable excipients for modulating the drug release profile such as medium chain triglycerides, poly(oxyethylene) sorbitan fatty acid esters (e.g. polysorbate 20, polysorbate 80), sorbitan fatty acid esters, cyclodextrins, lecithin, mannitol, sucrose, inorganic salts and mixtures thereof.

The polymeric matrix of the microparticles may comprise one or more excipient(s). Said excipient(s) may be present in the polymer matrix in an amount not exceeding 15% by weight, in particular not exceeding 10% by weight, and more particularly not exceeding 5% by weight, with respect to the total weight of the polymeric matrix.

The present invention further relates to a formulation under the form of a powder comprising controlled release microparticles comprising colchicine and a polymeric matrix, wherein the microparticles have a mean particle size equal or greater than 10 µm, and wherein the polymeric matrix comprises at least a poly(lactic-co-glycolic acid) copolymer, at least a poly(caprolactone) or at least a mixture of poly(lactic-co-glycolic acid) copolymer and poly(caprolactone), wherein the percentage weight ratio between the colchicine and the total weight of the microparticles ranges from 1 to 35% by weight, in particular from 2 to 30% by weight, more particularly between 5 and 25% by weight, and even more particularly between 10 and 20% by weight.

According to said aspect of the invention, the powder further comprises an immediate release dosage form containing colchicine, in particular wherein the maximal weight ratio of immediate release colchicine relative to the total amount of colchicine is 25%.

The present invention further relates to a pharmaceutical composition under the form of a sterile and injectable suspension suitable for an intra-articular injection, obtained by mixing a formulation under the form of a powder as described above, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

In one embodiment, said pharmaceutical composition is characterized by the concentration in colchicine ranging from 0.05 to 5 mg per ml of suspension, in particular ranging from 0.1 to 4 mg per ml of suspension, more particularly from 0.25 to 2.5 mg per ml of suspension, and even more particularly from 0.5 to 1.5 mg per ml of suspension.

### Manufacturing process for the obtention of the microparticles comprising a polymer matrix

Any process suitable to produce polymeric microparticles with a mean particle size ranging from 10 µm to 100 µm is considered appropriate in the framework of the present invention.

Among such manufacturing process emulsification-based processes, such as high-pressure homogenization, for example using rotor-stator homogenizer in a batch or continuous mode, or membrane emulsification, followed by organic solvent removal by extraction/evaporation may be cited.

As far as the general principal of such methods is concerned, an emulsion may be prepared and processed over a membrane with pores with a determined size. The obtained microspheres can then be collected after extraction and evaporation of the organic solvent, washed and freeze-dried.

According to one particular embodiment, the microparticles may be manufactured from an O/W direct emulsion or from a W/O/W double emulsion technique.

According to one particular embodiment, the microparticles may be manufactured from a Solid-in-Oil-in-Water (S/O/W) double emulsion technique.

Schoubben, A., Ricci, M. & Giovagnoli, S. Meeting the unmet: from traditional to cutting-edge techniques for poly lactide and poly lactide-co-glycolide microparticle manufacturing. J. Pharm. Investig. 49, 381-404 (2019) reviews various methods to make PLGA microparticles that can be used in the framework of the present invention.

Piacentini, E. Pharmaceutical Particles Design by Membrane Emulsification: Preparation Methods and Applications in Drug Delivery. Curr. Pharm. Des. 23, 302-318 (2018) reviews various designs of such membrane emulsification technique, which may be used in the framework of the present invention.

Other manufacturing approaches suitable for obtaining the microparticles according to the present invention are atomization by spinning disk, atomization by spray-drying, a fluidized bed coating, or combinations thereof.

Alternatively, the microparticles may be manufactured using drop-on-demand, drop-by-drop and jet break-up processes such as inkjet printing or microfluidics.

Alternatively, the microparticles may be manufactured using supercritical fluid technologies.

Alternatively, the microparticles may be manufactured using microfabrication methods, such as template and mold-based techniques, such as soft lithography.

All these manufacturing processes are well known by the man skilled in the art.

The hereabove cited manufacturing processes are well known to the man skilled in the art.

### (ii) MULTI VESICULAR LIPOSOMES

Multivesicular liposomes (MVL) are spherical particles with a mean diameter of 10-30 µm and composed of non-concentric multiple lipid bilayers arranged in a honeycomblike structure. These lipid layers enclose numerous water-filled aqueous compartments that can be used to encapsulate a water-soluble drug such as colchicine.

MVLs are classically composed of at least one amphiphilic lipid and one neutral lipid. The amphiphilic lipid is selected from phospholipids for instance phosphatidylcholine or phosphatidylglycerol. The neutral lipid is selected from triglycerides having mono-unsaturated fatty acid ester moieties containing 14-18 carbons in the acyl chain (e.g. triolein, tripalmitolein), saturated fatty acid ester moieties containing 6 to 8 carbons in the acyl chain (e.g. tricaproin, tricaprylin) and mixtures thereof. Cholesterol can also be used in the composition.

MVLs are obtained by using a water-in-oil-in-water double emulsification process. In a first step, a water-in-oil emulsion is prepared by mixing phospholipids, triolein, tricaprylin and cholesterol solubilized in volatile and water-immiscible organic solvent with an aqueous solution containing the solubilized drug to be encapsulated. This first emulsion is then emulsified by mixing with a second aqueous solution to produce the water-in-oil-in-water emulsion. The energy needed to form the first and second emulsion can be provided either mechanically, by sonication or by combinations thereof. MVLs are finally obtained by removal of the volatile organic solvent from the double emulsion using gas stripping or flushing. Finally, removal of unencapsulated material, concentration of the MVLs and buffer exchange is performed using either diafiltration or cross-flow filtration system.

In one embodiment, the neutral lipids used to manufacture the MVL encapsulating colchicine comprises a mixture of triolein : tricaprylin with ratios ranging from 50:50 to 0:100.

### PHARMACEUTICAL COMPOSITION

The pharmaceutical composition used in the framework of the present invention may take the form of a sterile and injectable composition, in particular a suspension composition, comprising an effective amount of colchicine.

By "sterile", in the sense of the present invention, is meant an environment capable of guaranteeing to the considered compounds in a composition according to the invention safety requirements for the administration routes such as above-mentioned, notably into or through a joint. Indeed, for obvious reasons, it is essential that a composition according to the invention be devoid of any contaminant body capable of initiating an undesirable side reaction at the host site.

The pharmaceutical composition used in the framework of the present invention may be prepared with the formulation under the form of a powder comprising microparticles as described above. According to one embodiment, said pharmaceutical composition is a sterile and injectable composition for controlled release of colchicine, suitable for an intra-articular injection.

By its injectable character, a composition according to the invention necessarily comprises a physiologically acceptable medium, also called "aqueous injection vehicle".

A "physiologically acceptable medium" means a medium devoid of toxicity and compatible with the injection and/or the application of the composition such as considered in the present invention.

The present invention more particularly relates to the pharmaceutical composition for use as defined above, wherein it is obtained by mixing a formulation under the form of a powder comprising controlled release microparticles comprising colchicine having a mean particle size equal or greater than 10 µm, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

The composition may comprise a solvent or a mixture of physiologically acceptable solvents.

The composition may comprise a physiologically acceptable aqueous medium.

As an aqueous medium suitable for the invention, may be for example mentioned water.

As isotonic agents suitable for the preparation of a composition according to the invention, it may be mentioned sugars and sodium chloride.

The aqueous injection vehicle may in particular comprise a tonicity-enhancing agent, a wetting-agent, a viscosity-enhancing agent or a mixture thereof.

Among the tonicity-enhancing agent, the following may be cited: dextrose, mannitol, sorbitol, sucrose, glycerin, sodium chloride, potassium chloride, cyclodextrin and maltodextrin.

Among the wetting agents, the following may be cited: poly(oxyethylene) sorbitan fatty acid esters, such as commercially available under the trade name TWEEN, sorbitan fatty acid esters, such as commercially available under the trade name SPAN, poloxamer and lecithins.

Among the viscosity-enhancing agents, the following may be cited: sodium carboxymethyl cellulose (CMC), a glycosaminoglycan such as hyaluronic acid, dextran, collagen, poly(vinylpyrrolidone), poly(ethyleneglycol), gelatin, hydroxyethyl cellulose (HEC), methyl cellulose (MC), alginate, gum acacia, starch.

According to a particular embodiment, said pharmaceutical composition has a viscosity of from 5 to 200 mPa.s, in particular from 5 to 100 mPa.s, and more particularly from 5 to 50 mPa.s.

According to one embodiment, said pharmaceutical composition may further comprise an immediate release dosage form containing colchicine. The presence of such immediate release dosage form aims at achieving a quick articular colchicine concentration so as to relieve pain quickly. In one embodiment, the maximal weight ratio of immediate release colchicine relative to the total amount of colchicine is 25%.

In certain embodiment of the invention, the weight ratio of immediate release colchicine relative to the total amount of colchicine may be between 0 and 5%, 0 and 10%, 0 and 15% or 0 and 20%.

In some embodiments, the length of release of the immediate release form is between 0 and 6 hours. In some embodiment, the length of release of the immediate release form is between 0 and 2 hours.

The immediate release dosage form may take the form of immediate fraction that are mixed with the controlled release microparticles as described above or be present in a continuous phase to which the microparticles are mixed when preparing the final formulation under the form of a powder, according to the present invention. Said continuous phase may of course comprise any further suitable pharmaceutically acceptable excipient than the ones already present within the microparticles.

### Additional active ingredients

Depending upon the particular condition, or disease, to be treated, additional therapeutic agents, which are normally administered to treat that condition, may be administered in combination with colchicine.

As used herein, the term "combination", "combined," and related terms refers to the simultaneous or sequential administration of said additional active ingredient with colchicine. For example, a combination may be administered with an additional active ingredient simultaneously or sequentially in separate unit dosage forms or together in a single unit dosage form.

According to one embodiment, hyaluronic acid may be injected into the painful joint, and for example be present in the powder or pharmaceutical composition according to the present invention. The use of hyaluronic acid in the treatment of arthritis, such as osteoarthritis, is well known, as viscosupplementation. Therefore, according to one embodiment, the present invention provides a pharmaceutical composition comprising colchicine according to the present invention further comprising hyaluronic acid.

In some embodiments, the present invention provides a pharmaceutical composition comprising colchicine according to the present invention further comprising at least an additional therapeutic agent. Suitable additional active ingredients are described in further detail below.

According to one embodiment, the following active ingredients may be combined, in particular any anti-inflammatory active ingredient:
- (i) corticosteroid, such as prednisone, prednisolone, methylprednisolone, betamethasone, dexamethasone, triamcinolone acetonide, triamcinolone hexacetonide, budenoside, mometasone, ciclesonide, fluticasone, hydrocortisone, and pharmaceutically acceptable salts thereof, especially at low dose,
- (ii) an NSAID, such as aspirin, diclofenac, aceclofenac, sulindac, ketorolac, ibuprofen, ketoprofen, naproxen, oxaprozine, flubiprofen, indomethacin, proglumetacin, tiaprofenic acid, meloxicam, piroxicam, tenoxicam, etodolac and celecoxib, etoricoxib, parecoxib, rofecoxib, valdecoxib, and pharmaceutically acceptable salts thereof,
- (iii) an antiIL-1β agents such as anakinra, canakinumab, rilonacept, and pharmaceutically acceptable salts thereof,
- (iv) Anti-IL-6 agents such as tocilizumab, siltuximab, sarilumab, and pharmaceutically acceptable salts thereof,
- (v) an anti-TNFα agent, such as adalimumab, etanercept, infliximab, certolizumab, golimumab, and pharmaceutically acceptable salts thereof,
- (vi) an anti-angiogenic agents such as bevacizumab, and pharmaceutically acceptable salts thereof,
- (vii) an anti NGF (Nerve Growth Factors) agent, such as fasinumab, tanezumab, fulranumab, ABT-110, and pharmaceutically acceptable salts thereof,
- (viii) an opioid, such as fentanyl, morphine, buprenorphine, hydromorphone, hydrocodone, oxycodone, meperidine, and pharmaceutically acceptable salts thereof,
- (ix) an inhibitor of class I glucose transporters (e.g. GLUT-1/GLUT-3), such as cytochalasin B, WZB-117, STF-31, BAY-876
- (x) mixtures thereof.

Said additional active ingredient may be formulated under immediate and/or controlled release dosage forms.

### Administration of the composition

A pharmaceutical composition used in the framework of the present invention can be injected using any of the known methods in the art.

Particularly, said pharmaceutical composition may be administered by means of an injection device suitable for intra-articular injection such as a syringe equipped with a needle 19-29G, preferentially 22-29G, more preferentially 25-29G.

Throughout the description, including the claims, the expression "comprising a" should be understood as being synonymous with "comprising at least one" unless specifically stated otherwise.

The expressions "between... and ..." and "ranging from ... to ..." should be understood to mean that the limits are inclusive, unless specified otherwise.

The following examples and figures are presented by way of non-limiting illustration of the invention.

### EXAMPLES

### Example 1: Sustained-release Colchicine microparticles

An organic solvent phase was prepared by solubilizing 0.23 g of colchicine (Indena, Italy) and 0.91 g of PLGA Resomer RG 503H (Evonik Industries AG, Essen, Germany) in 2.51 g of dichloromethane (Merck) under vortexing.

Emulsification step was performed by adding 5 g of a PVA solution at 1% (Mowiol 4-88, Sigma-Aldrich) to the organic phase under high shear emulsification (Heidolph homogenizer DIAX 900, 11600 rpm) during 1 minute.

Emulsion was then poured into a hardening bath (0,5 L of an aqueous PVA solution at 1%) and stirred for 3 hours at 300 rpm with an overhead stirrer.

The solid particles were then separated from aqueous phase by pouring the emulsion over a stainless-steel screen with 12 µm pore diameter and washed three times with 50 mL of distilled water.

The microparticles were then transferred to glass dishes, dried at 5°C under 7 mbar pressure for 18 hours and stored in amber glass vials.

### Analytical methods

Particle size was determined using optical microscopy (N=100 particles analyzed, Olympus BX51 with Stream Software).

Microparticle drug load was determined by dissolving 30 mg of particles with 2,5 mL acetonitrile in a 50 mL volumetric flask. After dissolution, 22,5 mL of methanol is added and mixed under vortexing. The flask was then filled up with water and thoroughly mixed using vortex. The medium was then centrifuged for 30 min at 4000 rpm and the supernatant is filtrated through a 0.45 µm PTFE filter (Acrodisc wwPTFE), discarding the first 2 mL. An aliquot of this solution is then analyzed by HPLC to determine the microparticle drug load.

Analysis by HPLC was conducted using a silica-based reversed-phase C8 column [GL Sciences, Inertsil C8-3, 4.6 × 250 mm] and a mobile phase [55% methanol, 45% of a 6.8 g/L KH₂PO₄ solution, solution adjusted to pH 5.5 with diluted phosphoric acid] at 1 mL/min flow rate with UV detection at 254 nm.

For the *in vitro* release tests from microparticles, aliquots of 10-30 mg microparticles were suspended in 50 mL of phosphate buffer (50 mM, pH 7.4) containing 0.02% sodium azide maintained at 37°C in a shaking incubator with orbital motion (GFL model 3033). At different intervals, 3 mL of the media was removed and replaced with an equivalent amount of fresh media, and analyzed by UV assay at 350 nm.

The results are shown in Table 3.

**Table 3. Analytical results for Colchicine PLGA microparticles**

| Drug loading content (colchicine wt %) | Particle size (Dv, µm) | *In vitro* release (%) |
|---|---|---|
| 12 | D10 = 20 µm | 1 day: 8.4 |
| | D50 = 35 µm | 2 days: 15 |
| | D90 = 87 µm | 3 days: 24.3 |
| | | 7 days: 73 |
| | | 10 days: 89.8 |
| | | 13 days: 90.4 |

In this example, colchicine-loaded PLGA microparticles were prepared with a mean particle size of 35 µm and releasing 80% of its drug content after 7 days.

### Example 2: Sustained-release Colchicine microparticles

An organic solvent phase was prepared by solubilizing 0.7 g of colchicine (Indena, Italy) and 1.05 g of sterile PLGA Resomer RG 503H (Evonik Industries AG, Essen, Germany) in 2.5 g of dichloromethane (Merck) under vortexing.

Emulsification step was performed in a laminar flow hood by adding 5 g of a sterile PVA solution at 1% (Mowiol 4-88, Sigma-Aldrich) to the organic phase under high shear emulsification (Heidolph homogenizer DIAX 900, 11600 rpm) during 1 minute.

Emulsion was then poured into a hardening bath (0,5 L of an aqueous PVA solution at 1% prepared using sterile water for injection) and stirred for 3 hours at 300 rpm with an overhead stirrer.

The solid particles were then separated from aqueous phase under laminar flow by pouring the emulsion over a stainless-steel screen with 12 µm pore diameter and washed three times with 50 mL of sterile water for injection.

The microparticles were then transferred to glass dishes, dried at 5°C under 7 mbar pressure for 18 hours and stored in sealed amber glass vials at 5°C.

Analytical tests were performed as in Example 1 and are shown in Table 4.

**Table 4. Analytical results for Colchicine PLGA microparticles**

| Drug loading content (colchicine wt %) | Particle size (Dv, µm) | *In vitro* release (%) |
|---|---|---|
| 26 | D10 = 11 µm | 0.13 day: 19.7 |
| | D50 = 17 µm | 1 day: 45.8 |
| | D90 = 25 µm | 2 days: 56.1 |
| | | 5 days: 85.7 |
| | | 8 days: 94.4 |

In this example, colchicine-loaded PLGA microparticles were prepared with a mean particle size of 17 µm and releasing 80% of its drug content between 2 days and 5 days.

### Example 3: Sustained-release Colchicine microparticles

An organic solvent phase was prepared by solubilizing 0.7 g of colchicine (Indena, Italy), 0.79 g of PLGA Resomer RG 503H and 0.26 g of PLGA Resomer RG 502H (Evonik Industries AG, Germany) in 2,52 g of dichloromethane (Merck) under vortexing.

Emulsification step was performed by adding 5g of a PVA solution at 1% (Mowiol 4-88, Sigma-Aldrich) to the organic phase under high shear emulsification (Heidolph homogenizer DIAX 900, 11600 rpm) during 1 minute.

Emulsion was then poured into a hardening bath (0,5 L of an aqueous PVA solution at 1%) and stirred for 3 hours at 300 rpm with an overhead stirrer.

The solid particles were then separated from aqueous phase by pouring the emulsion over a stainless-steel screen with 12 µm pore diameter and washed three times with 50 mL of distilled water.

The microparticles were then transferred to glass dishes, dried at 5°C under 7 mbar pressure for 18 hours and stored in amber glass vials.

Analytical tests were performed as in Example 1 and are shown in Table 5.

**Table 5. Analytical results for Colchicine PLGA microparticles**

| Drug loading content (colchicine wt %) | Particle size (Dv, µm) | *In vitro* release (%) |
|---|---|---|
| 19 | D10 = 14 µm | 0.13 day: 46 |
| | D50 = 32 µm | 1 day: 79.1 |
| | D90 = 44 µm | 2 days: 87 |

In this example, colchicine-loaded PLGA microparticles were prepared with mean particle size of 32 µm and releasing 80% of its drug content between 1 day and 2 days.

### Example 4: Sustained-release Colchicine microparticles - continuous emulsification using a high-shear in-line mixer

Microparticles were prepared using Silverson L5M-A high shear in-line mixing assembly (SILVERSON Machines, East Longmeadow, Massachusetts, U.S.A.) equipped with an emulsion stator screen with fine 0.8 mm perforations. Bottom inlet port of the mixing chamber was equipped with a male run tee connected to two different perilstatic pumps through a 1 mm diameter Viton tubing and a 5 mm diameter Viton tubing. Outlet port was equipped with a 5 mm diameter Viton tubing.

The dispersed phase was prepared by dissolving 25 g of PLGA copolymer (Resomer RG503H, Evonik Industries AG, Germany) and 4.4 g of colchicine in 100 g of ethyl acetate (Alfa Aesar).

Separately, a continuous phase was prepared by adding 74.9 g of ethyl acetate to 998 g of an aqueous solution containing 1wt% PVA.

For the emulsification step, the Silverson speed was set at 2000 rpm and the dispersed phase was fed through the 1 mm diameter inlet tube at a flow rate of about 20 g/min and the continuous phase was fed simultaneously through the 5 mm diameter inlet tube at a flow rate of about 200 g/min.

Resulting emulsion flowing from the outlet port was added to a beaker containing a 6 L water solution used to extract the organic solvent and that was being stirred at 300 rpm with a laboratory mixer.

After 2 hours of extraction duration, the resulting suspension was passed through a stainless-steel screen with 12 µm pore diameter, rinsed three times with 150 mL of distilled water and finally dried by lyophilization.

### Example 5: Pharmaceutical composition - Preparation of injectable suspension

An aqueous injection vehicle was prepared using pyrogen-free excipients and consisting of 1.4% of low viscosity sodium carboxymethyl cellulose (Aqualon CMC 7LF PH BET, Ashland), 0.1% Polysorbate 20 (Acros Organics), 0.13% disodium hydrogen phosphate dihydrate, 0.1% citric acid (Roth) and 0.65% sodium chloride (Roth). Final pH of the solution was adjusted to 7.2 using concentrated sodium hydroxide solution (Roth). The vehicle was then autoclaved at 121°C for 15 minutes (MultiControl 2, CertoClav) and 5 mL aliquots of the solution transferred aseptically into 10 mL vials under a laminar air flow bench.

A 20 mg aliquot of microparticles as prepared according to example 2 was dispersed in each vial using a vortex mixer and under a laminar flow.

This pharmaceutical composition is intended for intra-articular injection to treat crystal-associated and non-crystal associated acute inflammatory arthritis, in particular non-crystal associated acute arthritis, such as Rheumatoid arthritis, Ankylosing spondylitis, Juvenile arthritis, Psoriatic arthritis, Lupus or crystal-associated arthropathies attacks or flares, with an injection volume to be adapted to the particular intra-articular injection site.

In an example, a volume of 0.25 mL containing a 0.26 mg colchicine dose can be injected into the first metatarsophalangeal joint using a 27 G gauge needle.

In another example a volume of 2.5 mL containing a 2.59 mg colchicine dose can be injected into the knee joint using a 23 G gauge needle.

## Claims

1. A pharmaceutical composition comprising controlled release microparticles comprising colchicine, suitable for intra-articular injection into a joint, for use in the treatment of crystal-associated and non-crystal associated acute inflammatory arthritis, in particular a crystal-associated arthropathy or flares or acute forms of tendinitis and capsulitis, wherein the microparticles have a mean particle size greater than 10 µm, wherein the time required to release 80% by weight of the colchicine in the microparticles lies between 1 day and 15 days, wherein the colchicine is present in a concentration ranging from 0.05 to 5 mg per ml of suspension and wherein the pharmaceutical composition has a volume ranging from 0.1 ml to 5 ml.

2. The pharmaceutical composition for use according to claim 1, wherein the colchicine is present in a concentration ranging from 0.1 to 4 mg per ml of suspension, in particular from 0.25 to 2.5 mg per ml of suspension, and more particularly from 0.5 to 1.5 mg per ml of suspension.

3. The pharmaceutical composition for use according to claim 1 or 2, wherein the microparticles are microparticles comprising a polymeric matrix or multivesicular liposomes.

4. The pharmaceutical composition for use according to anyone of claims 1 to 3, wherein the microparticles are microparticles comprising colchicine and a polymeric matrix, wherein the polymeric matrix comprises at least a poly(lactic-co-glycolic acid) copolymer, at least poly(caprolactone) or at least a mixture of poly(lactic-co-glycolic acid) copolymer and poly(caprolactone).

5. The pharmaceutical composition for use according to anyone of claims 1 to 4, wherein the crystal-associated and non-crystal associated acute inflammatory arthritis is selected from non-crystal associated acute arthritis, such as Rheumatoid arthritis, Ankylosing spondylitis, Juvenile arthritis, Psoriatic arthritis, Lupus and crystal-associated arthropathy attacks or flares or acute forms of tendinitis and capsulitis, and more particularly selected from gout, chondrocalcinosis, calcifying tendinitis and Milwaukee shoulder syndrome.

6. The pharmaceutical composition for use according to anyone of claims 1 to 5, wherein the joint is selected from the metatarsophalangeal joints, the metacarpophalangeal joints, the knee joint, the shoulder joint, the wrist joint, the elbow joint, the ankle joint, the hip joint, a vertebral joint and the midfoot joints.

7. The pharmaceutical composition for use according to anyone of claims 1 to 6, wherein the time required to release 80% by weight of the colchicine in the microparticles lies between 1 day and 10 days, after 1 day to 7 days, after 1 day to 5 days, and for example after 1 day, 2 days, 3 days, 4 days, 5 days, 6 days, 7 days, 8 days, 9 days or 10 days.

8. The pharmaceutical composition for use according to anyone of claims 1 to 7, wherein the microparticles have a mean particle size lower than 100 µm, in particular lower than 80 µm, and more particularly lower than 50 µm, for example between 10 and 50 µm or between 15 and 40 µm.

9. The pharmaceutical composition for use according to anyone of claims 1 to 8, wherein it is obtained by mixing a formulation under the form of a powder comprising controlled release microparticles comprising colchicine having a mean particle size equal or greater than 10 µm, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

10. The pharmaceutical composition for use according to anyone of claims 1 to 9, wherein it has a viscosity of from 5 to 200 mPa.s, in particular from 5 to 100 mPa.s, and more particularly from 5 to 50 mPa.s.

11. The pharmaceutical composition for use according to anyone of claims 1 to 10, wherein it further comprises an immediate release dosage form containing colchicine, in particular wherein the maximal weight ratio of immediate release colchicine relative to the total amount of colchicine is 25%.

12. The pharmaceutical composition for use according to anyone of claims 4 to 11, wherein the polymeric matrix further comprises one or more additional polymer(s) or copolymer(s) selected from poly(lactide), poly(glycolide), poly(lactide-co-caprolactone), poly(ethylene glycol), poly(ethylene oxide), PLGA-b-PEO-b-PLGA, PLGA-b-PEO, polyhydroxyalkanoates, poly(hydroxybutyrate), poly(trimethylene carbonate), poly(dioxanone), poly(valerolactone), poly(alpha- hydroxy acids), poly(lactones), poly(amino acids), polyanhydrides, poly(orthoester), poly(acetals), polyurethanes, polythioesters, polyphosphoesters, poly(ester-co-amide), poly(vinyl alcohol), PVA-g-PLGA, poly (ether ester) multiblock copolymers, polyvinyl pyrrolidone, poly(methacrylate), PEO-PPO-PEO, gelatin, heparin, chondroitin sulfate; polysaccharides such as alginates, starch, chitosan and dextrans and any combinations thereof, and in particular selected from poly(lactide) and poly(caprolactone).

13. The pharmaceutical composition for use according to anyone of claims 4 to 12, wherein the polymeric matrix comprises poly(lactic-co-glycolic acid), copolymer poly(caprolactone) or a mixture of poly(lactic-co-glycolic acid) copolymer and poly(caprolactone) in an amount of greater than 70% by weight, with respect to the total weight of the polymeric matrix, in particular greater than 80% by weight, and even more particularly greater than 90% by weight.

14. The pharmaceutical composition for use according to anyone of claims 1 to 13, wherein, the drug loading content or percentage weight ratio between the colchicine and the total weight of the microparticles ranges from 1% to 35%, in particular from 2 to 30%, even more particularly from 5 to 25%, for example from 10 to 20%.

15. The pharmaceutical composition for use according to anyone of claims 1 to 14, wherein the volume is adapted to the joint, and namely ranges from 2 to 5 ml for the shoulder, from 1 to 2 ml for a vertebral joint, from 0.1 to 1 ml for the midfoot joints, from 0.1 to 0.5 ml for the metatarsophalangeal joints, from 0.1 to 0.5 ml for the metacarpophalangeal joints, from 2 to 5 ml for the knee joint, from 0.5 to 1 ml for the wrist joint, from 2 to 5 ml for the elbow joint, from 1 to 3 ml for the ankle joint and from 2 to 5 ml for the hip.

16. A formulation under the form of a powder comprising controlled release microparticles comprising colchicine and a polymeric matrix, wherein the microparticles have a mean particle size equal or greater than 10 µm, and wherein the polymeric matrix comprises at least a poly(lactic-co-glycolic acid) copolymer, at least a poly(caprolactone) or at least a mixture of poly(lactic-co-glycolic acid) copolymer and poly(caprolactone), wherein the percentage weight ratio between the colchicine and the total weight of the microparticles ranges 1 to 35% by weight, in particular from 2 to 30% by weight, more particularly between 5 and 25% by weight, and even more particularly between 10 and 20% by weight.

17. The formulation according to claim 16, wherein the powder further comprises an immediate release dosage form containing colchicine, in particular wherein the maximal weight ratio of immediate release colchicine relative to the total amount of colchicine is 25%.

18. A pharmaceutical composition under the form of a sterile and injectable suspension suitable for an intra-articular injection, obtained by mixing a formulation under the form of a powder according to the claim 16 or 17, with an aqueous injection vehicle, wherein the pharmaceutical composition optionally comprises an excipient selected from a group consisting of a tonicity-enhancing agent, a wetting-agent, a viscosity-enhancing agent, a density-enhancing agent or a mixture thereof, and wherein said excipient may be present in the aqueous injection vehicle or in the powder.

19. A pharmaceutical composition according to claim 18, wherein the colchicine is present in a concentration ranging from 0.05 to 5 mg per ml of suspension, in particular ranging from 0.1 to 4 mg per ml of suspension, more particularly from 0.25 to 2.5 mg per ml of suspension, and even more particularly from 0.5 to 1.5 mg per ml of suspension.
